# EUROPEAN PATENT APPLICATION

(11) **EP 4 516 230 A1**
(43) Date of publication of application: **05.03.2025**
(21) Application number: 23796809.4
(22) Date of filing: 27.04.2023
(51) Int. Cl.: A61B 6/00

(54) **X-RAY IMAGING APPARATUS**

(30) Priority: 27.04.2022 KR 20220051797
(71) Applicant: Rayence Co., Ltd., Hwaseong-si, Gyeonggi-do 18449 (KR); Vatech Ewoo Holdings Co., Ltd., Hwaseong-si, Gyeonggi-do, 18449 (KR)
(72) Inventor: YOON, Jongbo, Hwaseong-si, Gyeonggi-do 18449 (KR); KIM, Byeongnyeon, Hwaseong-si, Gyeonggi-do 18449 (KR)
(74) Representative: Lorenz & Kollegen
(86) International application number: PCT/KR2023/005730
(87) International publication number: WO 2023/211166

(57) **Abstract**

The present invention relates to an X-ray imaging apparatus having an X-ray generator and an X-ray detector disposed at both ends of a C-shaped arm so as to take X-ray images of an object placed therebetween, the X-ray imaging apparatus comprising: the C-shaped arm; a guide rail connected to one end of the C-shaped arm; the X-ray generator connected to the guide rail; the X-ray detector disposed at the other end of the C-shaped arm and facing the X-ray generator; and a drive unit for moving the X-ray generator along the guide rail.

## Description

### Technical Field

The present disclosure relates to an X-ray imaging apparatus in which an X-ray generator and an X-ray detector are disposed at opposite ends of a C-shaped arm so as to take X-ray images of an object placed therebetween.

### Background Art

An X-ray machine is a device that emits X-rays to an object being x-rayed, detects the X-rays that travel through the object, and provides an X-ray image of the internal structure of the object.

An example of the X-ray machine is an X-ray fluoroscopy apparatus, commonly called a C-arm (mobile C-arm), used for surgery, procedures, or other clinical purposes. As an example, the C-arm X-ray machine consists of a movable mobile base, a multi-joint arm supporter connected to the mobile base, and a C-shaped arm connected to the arm supporter. An X-ray generator and an X-ray detector are installed at opposite ends of the C-shaped arm, respectively, to take an X-ray fluoroscopy image or an X-ray projection image of an object placed therebetween.

The C-arm X-ray machine is mainly used to obtain two-dimensional X-ray fluoroscopy images or X-ray projection images of an object, but in order to determine the 3D location of a surgical site or lesion, a limited angle tomography function such as tomosynthesis is required. To this end, the X-ray generator needs to rotate and move relative to an object being x-rayed to obtain multiple projection images in a certain angle range for the object.

However, in a typical C-arm X-ray machine, because an X-ray generator is fixed to one end of the C-shaped arm, the entire C-shaped arm needs to be rotated to obtain multiple projection images in a certain angle range. Therefore, a complex driving mechanism is required, the shooting time is long, precise control is difficult, and the complicated driving mechanism may result in increased load and footprint.

### [Document of Related Art]

### [Patent Document]

(Patent Document 1) Korean Patent Application Publication No. 10-2016-0071938
(Patent Document 2) Korean Patent Application Publication No. 10-2013-0058633
(Patent Document 3) U.S. Patent Application Publication No. 2020-0085390

### Disclosure

### Technical Problem

The present disclosure is intended to solve the above problems occurring in the related art. An objective of the present disclosure is to provide an X-ray imaging apparatus in which an X-ray generator can rotate and move separately with respect to a C-shaped arm while facing an X-ray detector.

### Technical Solution

In order to achieve the above mentioned objectives, there is provided an X-ray imaging apparatus including: a C-shaped arm; a guide rail connected to a first end of the C-shaped arm; an X-ray generator connected to the guide rail; an X-ray detector disposed at a second end of the C-shaped arm and configured to face the X-ray generator; and a drive part configured to move the X-ray generator along the guide rail.

At this time, the X-ray generator may move along the guide rail while maintaining an equal distance from a point of the X-ray detector.

In addition, the X-ray imaging apparatus may further include: a movable mobile base; and an arm supporter configured to connect the mobile base and the C-shaped arm.

In addition, the X-ray generator and the X-ray detector may capture at least one of an X-ray fluoroscopy image, an X-ray projection image, a limited angle tomographic image, an angle X-ray fluoroscopy image, and an angle X-ray projection image of an object placed between the X-ray generator and the X-ray detector.

### Advantageous Effects

According to the X-ray imaging apparatus of the present disclosure, the following effects are achieved.

Since the X-ray generator can be rotated and moved separately with respect to the C-shaped arm, the imaging time required for limited angle tomography can be short, precise control can be possible, and the overall driving mechanism can be simplified, which can lead to reduced load and footprint.

### Description of Drawings

FIG. 1 is a front view showing an X-ray imaging apparatus according to an embodiment of the present disclosure.
FIGS. 2 to 5 are detailed views of a portion of an X-ray imaging apparatus according to an embodiment of the present disclosure.
FIG. 6 is a cross-sectional view taken along line A-A in FIG. 4.

### Best Mode

Embodiments disclosed in the present specification will be described in detail with reference to the attached drawings, and identical or similar components will be assigned the same reference numbers regardless of the reference numerals in the drawing, and redundant descriptions thereof will be omitted. The suffixes "module" and "part" for components used in the following description are given or used interchangeably only for the ease of preparing the specification, and do not have distinct meanings or roles in themselves. In describing embodiments disclosed herein, if it is determined that a detailed description of related known technologies may unnecessarily obscure the gist of the present disclosure, the detailed description thereof will be omitted. In addition, the attached drawings are only for easy understanding of the embodiments disclosed in this specification, and it should be understood that the technical idea disclosed in this specification is not limited by the attached drawings, but includes all changes, equivalents, and substitutes included in the spirit and technical scope of the present disclosure.

Terms containing ordinal numbers, such as first, second, etc., may be used to describe various components, but the components are not limited by the terms, and the terms are used only for the purpose of distinguishing one component from another.

When a component is said to be "connected" to another component, it should be understood that the component may be directly connected to another component, but another component may exist in between. On the other hand, when a component is referred to as being "directly connected" to another component, it should be understood that there are no other components in between.

Singular expressions include plural expressions unless the context clearly indicates otherwise.

In this application, it should be understood that terms such as "comprise" or "have" are intended to designate that a feature, number, step, operation, component, part, or combination thereof described in the specification exists, and do not preclude the possibility of addition or existence of one or more other features or numbers, steps, operations, components, parts, or combinations thereof.

In the drawings, the sizes of components may be exaggerated or reduced for convenience of explanation. For example, the size and thickness of each component shown in the drawings are arbitrarily shown for convenience of explanation, so the present disclosure is not necessarily limited to what is shown.

In cases where an embodiment may be implemented differently, a specific process sequence may be performed differently from the described sequence. For example, two processes described in succession may be performed substantially at the same time, or may be performed in the reverse order from the order in which they are described.

In the drawings, the x-axis refers to a front-back direction, the y-axis refers to an up-down direction, and the z-axis refers to a left-right direction.

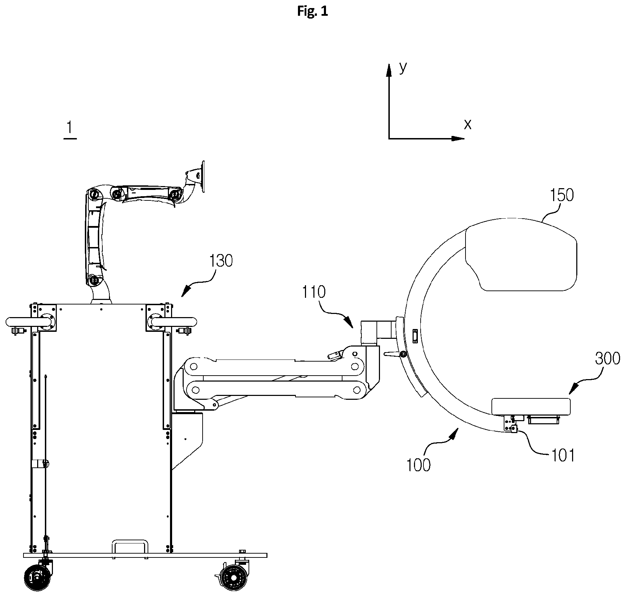

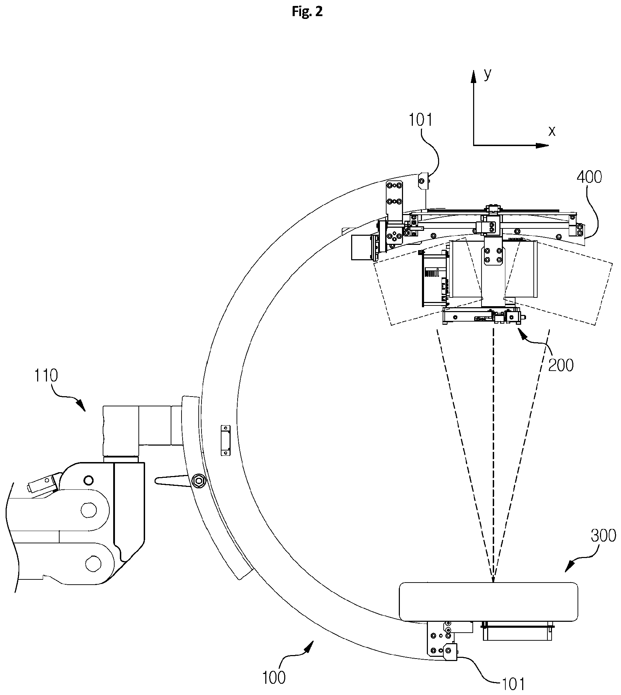

FIG. 1 is a front view showing an X-ray imaging apparatus 1 according to an embodiment of the present disclosure, and FIGS. 2 to 5 are detailed views of a portion of an X-ray imaging apparatus according to an embodiment of the present disclosure.

Referring to FIGS. 1 to 3, the X-ray imaging apparatus 1 according to an embodiment of the present disclosure may be a C-arm X-ray imaging device including a C-shaped arm 100. The C-shaped arm 100 may include a C-shaped or U-shaped curved segment, so that the cross-section is C-shaped or U-shaped.

The C-shaped arm 100 may be supported on an arm supporter 110. The C-shaped arm 100 may slide along the arm supporter 110. The C-shaped arm 100 may rotate with respect to the arm supporter 110. The arm supporter 110 may have at least one arm connected by a joint structure.

The arm supporter 110 may be installed on a mobile base 130. The mobile base 130 may travel and may include at least one wheel for this purpose. The mobile base 130 may be equipped with a controller (not shown) such as a computer device. The controller (not shown) may use detection results of an X-ray detector 300 to reconstruct an X-ray fluoroscopy image, an X-ray projection image, and a limited angle tomographic image and display the images on a monitor (not shown) installed on the mobile base 130.

An X-ray generator casing 150 may be installed at one end of the C-shaped arm 100.

The X-ray detector 300 may be installed at the other end of the C-shaped arm 100. The X-ray detector 300 may be built into an X-ray detector casing (not shown) installed at the other end of the C-shaped arm 100.

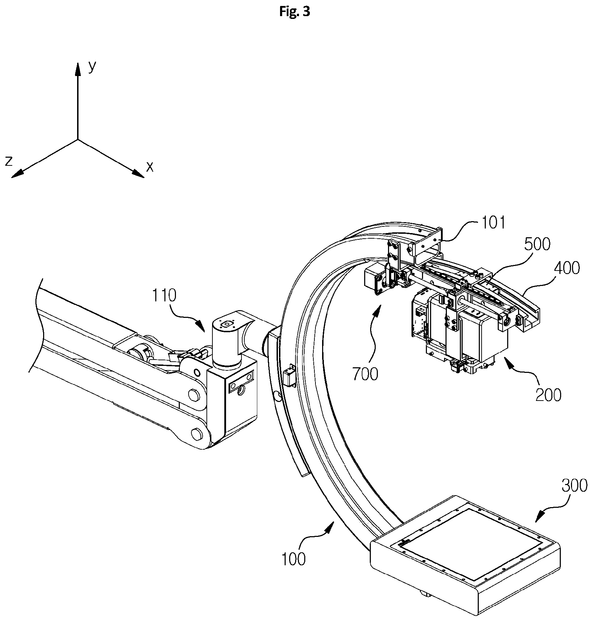

Referring to FIGS. 2 and 3, the X-ray imaging apparatus 1 according to an embodiment of the present disclosure may include an X-ray generator 200. The X-ray generator 200 may be built into the X-ray generator casing (150 in FIG. 1) along with a guide rail 400 and a driving part 700, which will be described later. In the drawings of FIG. 2 and thereafter, the X-ray generator casing is removed for convenience. The X-ray generator 200 may generate X-rays and emit the generated X-rays to the X-ray detector 300. The X-ray generator 200 may may be disposed at one end of the C-shaped arm 100. The X-ray generator 200 may include, for example, a field emission type X-ray source. The field emission type X-ray source may include a cathode electrode on which an emitter is installed, an anode electrode on which a target is installed, and a gate electrode interposed between the emitter and the target. The field emission type X-ray source may generate X-rays as electrons are emitted from the emitter by a gate voltage applied to the gate electrode, and the emitted electrons are accelerated toward the anode electrode and collide with the target due to the voltage difference between cathode and anode voltages applied to the cathode and anode electrodes, respectively.

The X-ray imaging apparatus 1 according to an embodiment of the present disclosure may include the X-ray detector 300. The X-ray detector 300 may detect X-rays that have passed through an object to be examined. The X-ray detector 300 may be a digital X-ray detector.

Referring to FIGS. 3 to 5, the X-ray imaging apparatus 1 according to an embodiment of the present disclosure may include: a guide rail 400 connected to one end of the C-shaped arm; a moving block 500 capable of rotating and moving along the guide rail 400; an X-ray generator 200 connected to the moving block 500; and a drive part 700 that rotates and moves the moving block 500 along the guide rail 400 to rotate and move the X-ray generator 200 connected to the moving block 500 along the guide rail 400.

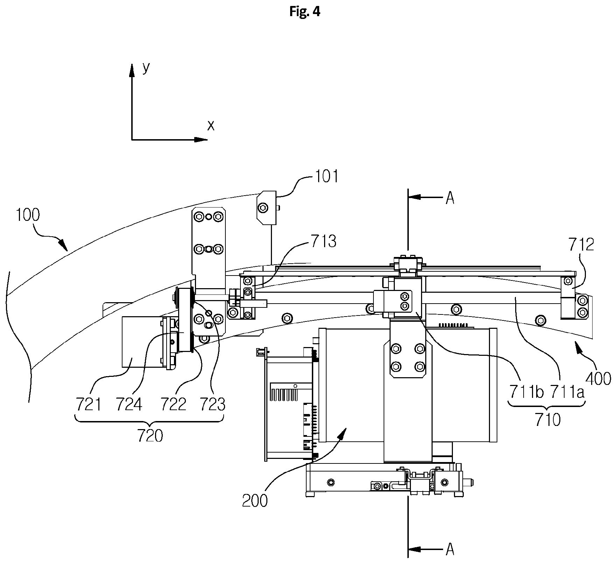

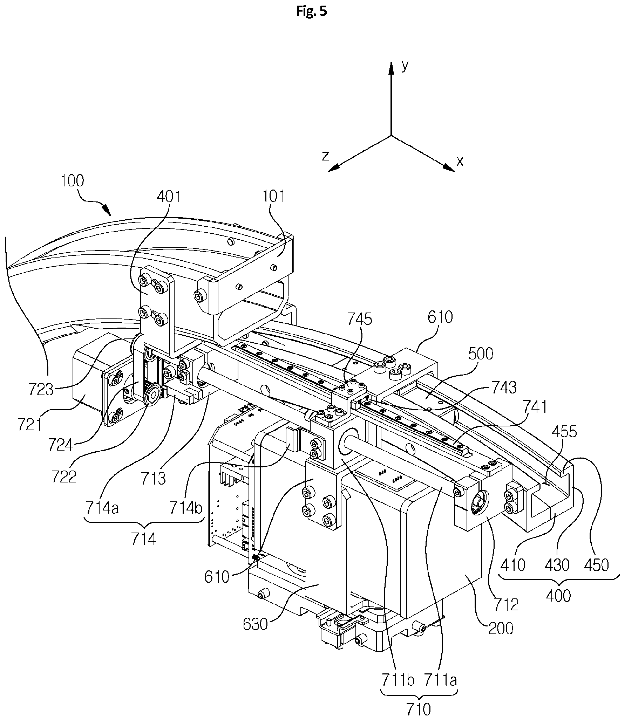

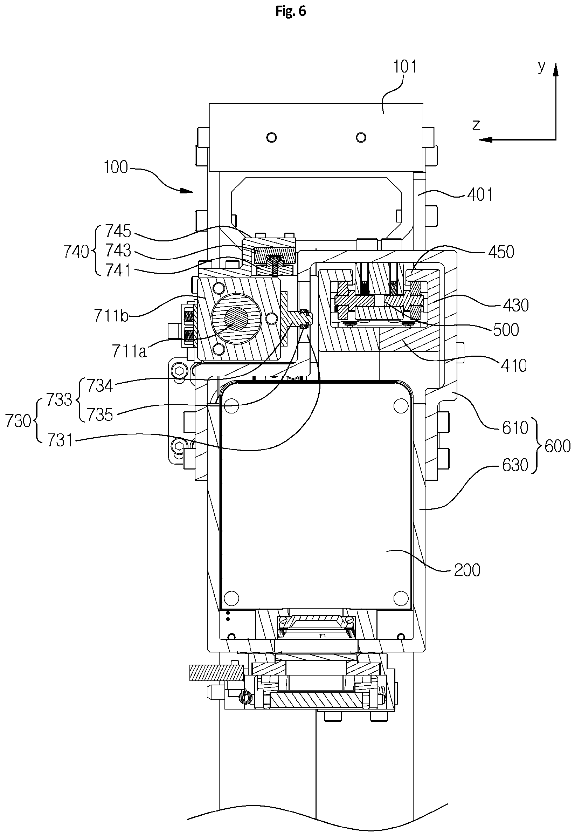

Referring to FIGS. 5 and 6, the guide rail 400 may have a circular arc or arch shape with the center thereof convex upward from opposite ends. FIG. 6 is a cross-sectional view taken along line A-A in FIG. 4. The guide rail 400 may have an arc shape equidistant from a point on the X-ray detector 300. The guide rail 400 may be connected to one end of the C-shaped arm 100 by means of a bracket 401. The guide rail 400 may include: a floor 410; side walls 430 provided on left and right sides of the bottom floor 410; and left and right locking protrusions 450 protrude from the left and right tops of the side walls 430 inward facing each other. The guide rail 400 may be provided with an opening 455 with open front and rear surfaces, and formed by the bottom floor 410, the side walls 430, and the arc-shaped left and right locking protrusions 450 along the longitudinal direction.

Referring to FIGS. 5 and 6, the moving block 500 may be movably placed inside the guide rail 400. The shape of the moving block 500 is not particularly limited as long as the moving block 500 can rotate and move along the guide rail 400 within the guide rail 400, that is, within the opening 455.

Referring to FIG. 6, the X-ray imaging apparatus 1 according to an embodiment of the present disclosure may include a moving bracket 600. The moving bracket 600 may connect the X-ray generator 200 and the moving block 500. The moving bracket 600 may include a first moving bracket 610 and a second moving bracket 630. The first moving bracket 610 may be connected to the moving block 500. The second moving bracket 630 may connect the first moving bracket 610 and the X-ray generator 200.

Referring to FIG. 5, the X-ray imaging apparatus 1 according to an embodiment of the present disclosure may include the drive part 700 that moves the moving block 500 along the guide rail 400.

The drive part 700 may include a motor 721, and a conversion part 710 that converts the rotational motion of the motor 721 into linear motion. In this embodiment, the conversion part 710 may include, for example, a lead screw 711a and a nut 711b.

The lead screw 711a may be rotatably supported by first and second bearing parts 712 and 713 installed before and after one side of the guide rail 400. The lead screw 711a may have threads formed on the outer peripheral surface thereof. The nut 711b may be fastened to the lead screw 711a. The nut 711b has threads formed on the inner peripheral surface thereof and may move linearly back and forth around the lead screw 711a according to the rotation direction of the lead screw 711a. The nut 711b may be connected to the moving block 500 via a lifting guide part 730, which will be described later.

The nut 711b may include a limit switch. The limit switch may be a switch that opens and closes an electrical contact when a part of a mechanical part or an object reaches a predetermined position. The limit switch may be composed of a mechanical limit switch, a beam-type limit switch, or a proximity switch. The limit switch of this embodiment may be configured as a proximity switch. Proximity switches may operate when an object approaches a certain distance using magnetism, capacitance, or electromagnetic induction. Referring to FIG. 5, a proximity switch 714 may include a first proximity switch 714a. The first proximity switch 714a may be installed in the first and second bearing parts 712 and 713. The proximity switch 714 may include a second proximity switch 714b. The second proximity switch 714b may be installed on the nut 711b. The second proximity switch 714b may be installed on the side of the nut 711b. When the first proximity switch 714a detects the second proximity switch 714b, the motor 721 is stopped and the nut 711b may be stopped.

The drive part 700 may include a power transmission part 720. The power transmission part 720 may provide rotational power of the motor 721 to the lead screw 711a. The power transmission part 720 may include a driving pulley 722. The driving pulley 722 may be connected to the rotation shaft of the motor 721. The power transmission part 720 may include a driven pulley 723. The driven pulley 723 may be connected to the lead screw 711a. The power transmission part 720 may include a belt 724. The belt 724 may connect the driving pulley 722 and the driven pulley 723. The belt 724 may be configured as a timing belt.

The drive part 700 may include the lifting guide part 730. The lifting guide part 730 may allow the moving block 500 to rotate and move along the guide rail 400 by the nut 711b moving along the lead screw 711a, while guiding the lifting and lowering of the moving block 500, which rotates and moves in an arc trajectory along the guide rail 400, so that the X-ray generator 200 rotates and moves in an arc trajectory along the guide rail 400.

Referring to FIG. 6, the lifting guide part 730 may include a guide groove 731 formed on the moving bracket 600. The guide groove 731 may be configured as a vertical slot extending upward and downward on one side of the first moving bracket 610. The lifting guide part 730 may include a guide pin part 733 installed on one side of the nut 711b. The guide pin part 733 may include a guide pin 734 movable along the guide groove 731 of the moving bracket 600, and a ring bearing 735 installed on the guide pin 734. Since the ring bearing 735 is rotatably installed with respect to the guide pin 734, the guide pin 734 may move along the up-down guide groove 731 of the moving bracket 600.

Thus, the interference between the circular movement of the moving block 500 by the guide rail 400 and the linear movement of the nut 711b by the lead screw 711a may be resolved, and the X-ray generator 200 may rotate and move along the guide rail 400 along an arc equidistant from a point on the X-ray detector 300. The point on the X-ray detector 300 may be the center of the X-ray detector 300.

The drive part 700 may include a linear guide part 740. The linear guide part 740 may guide the linear movement of the nut 711b. The linear guide part 740 may include an LM guide.

Referring to FIG. 6, the linear guide part 740 may include a linear guide rail 741. The linear guide rail 741 may be installed on the upper surfaces of the first and second bearing parts 712 and 713.

The linear guide part 740 may include a guide block 743. The guide block 743 may be installed on the linear guide rail 741. The guide block 743 may be slidably assembled to the linear guide rail 741.

The linear guide part 740 may include a bracket 745 connecting the nut 711b and the guide block 743.

The X-ray generator 200, the guide rail 400, and the drive part 700 as described above may be accommodated inside the X-ray generator casing 150 as shown in FIG. 1.

The X-ray generator 100 may rotate and move along the guide rail 400 within the X-ray generator casing 150 along an arc trajectory of the same distance with respect to a point on the X-ray detector. As a result, the X-ray imaging apparatus 1 according to an embodiment of the present disclosure may acquire a plurality of projection images in a certain angle range for an object being x-rayed. Therefore, the X-ray imaging apparatus 1 according to an embodiment of the present disclosure may provide a limited angle tomographic image, such as a tomosynthesis image, using the projection images in a certain angle range for the object being x-rayed.

In particular, according to the X-ray imaging apparatus 1 according to an embodiment of the present disclosure, the movement and rotation of the X-ray generator 200 are concealed by the X-ray generator casing 150, so the rotation and movement of the X-ray generator 200 cannot be seen from the outside, and problems such as a user running into the X-ray generator 200 may be prevented in advance.

In addition, in the drawings, the guide rail 400 of the X-ray imaging apparatus 1 according to the present disclosure is shown as being connected to one end of the C-shaped arm 100 along the longitudinal direction thereof. However, the guide rail 400 may also be connected to one end of the C-shaped arm 100 so as to intersect the longitudinal direction thereof, and in this case, the X-ray generator may rotate and move along a direction intersecting the longitudinal direction of the C-shaped arm 100 to perform limited angle tomography. Furthermore, the guide rail 400 may be connected to one end of the C-shaped arm 100 with a hinge and may change the arrangement direction thereof, and in this case, the X-ray generator may rotate and move along the arrangement direction of the guide rail 400 to perform limited angle tomography from various angles.

In addition, according to the X-ray imaging apparatus 1 according to an embodiment of the present disclosure, it is possible for the X-ray generator to move along the guide rail and stop at an appropriate point, and take an angle X-ray fluoroscopy image or an angle X-ray projection image tilted at a predetermined angle with respect to the object being x-rayed. That is, the X-ray imaging apparatus according to the present disclosure may capture at least one of an X-ray fluoroscopy image, an X-ray projection image, a limited angle tomographic image, an angle X-ray fluoroscopy image, and an angle X-ray projection image of the object placed between the X-ray generator and the X-ray detector, and provide X-ray images for each.

Any embodiments of the present disclosure described above are not exclusive or distinct from each other. In any embodiments of the present disclosure described above, individual configuration or function may be used in combination or combined.

It is obvious to those skilled in the art that the present disclosure may be embodied in other specific forms without departing from the spirit and essential features of the present disclosure. The above detailed description should not be construed as restrictive in any respect and should be considered illustrative. The scope of the present disclosure should be determined by reasonable interpretation of the appended claims, and all changes within the equivalent scope of the present disclosure are included in the scope of the present disclosure.

### [Description of Reference Numerals]

100: C-shaped arm 150: X-ray generator casing
200: X-ray generator 300: X-ray detector
400: guide rail 500: moving block
600: moving bracket 700: drive part
710: conversion part 720: power transmission part

## Claims

1. An X-ray imaging apparatus, comprising:
a C-shaped arm;
a guide rail connected to a first end of the C-shaped arm;
an X-ray generator connected to the guide rail;
an X-ray detector disposed at a second end of the C-shaped arm and configured to face the X-ray generator; and
a drive part configured to move the X-ray generator along the guide rail.

2. The apparatus of claim 1, wherein the X-ray generator moves along the guide rail while maintaining an equal distance from a point of the X-ray detector.

3. The apparatus of claim 1, wherein the guide rail is arched and maintains an equal distance from a point of the X-ray detector, and
the X-ray generator moves along the guide rail while maintaining an equal distance from the point of the X-ray detector.

4. The apparatus of claim 3, further comprising:
a moving block configured to move along the guide rail; and
a moving bracket configured to connect the moving block and the X-ray generator,
wherein the drive part moves the moving block along the guide rail to move the X-ray generator along the guide rail.

5. The apparatus of claim 4, wherein the drive part comprises:
a motor configured to generate rotational power; and
a conversion part configured to convert the rotational power into a linear motion in a longitudinal direction of the guide rail, and move the moving block along the guide rail using the linear motion.

6. The apparatus of claim 5, wherein the conversion part comprises:
a lead screw disposed along a front-rear direction of the guide rail and rotated by the motor;
a nut fastened to the lead screw and configured to move linearly along the lead screw by rotation of the lead screw; and
a lifting guide part configured to connect the nut and the moving block and guide lifting and lowering of the moving block in relation to the linear movement of the nut.

7. The apparatus of claim 1, further comprising:
a movable mobile base; and
an arm supporter configured to connect the mobile base and the C-shaped arm.

8. The apparatus of claim 1, wherein the X-ray generator and the X-ray detector captures at least one of an X-ray fluoroscopy image, an X-ray projection image, a limited angle tomographic image, an angle X-ray fluoroscopy image, and an angle X-ray projection image of an object placed between the X-ray generator and the X-ray detector.

9. The apparatus of claim 1, further comprising:
an X-ray generator casing configured to accommodate the guide rail, the X-ray generator, and the drive part thereinside.
